# EUROPEAN PATENT SPECIFICATION

(11) **EP 3 921 011 B1**
(45) Date of publication and mention of the grant of the patent: **01.05.2024**
(21) Application number: 20703089.1
(22) Date of filing: 22.01.2020
(51) Int. Cl.: A61M 25/06

(54) **MEDICAL DEVICE WITH MISALIGNED CANNULA**
MEDIZINISCHE VORRICHTUNG MIT FEHLAUSGERICHTETER KANÜLE
DISPOSITIF MÉDICAL À CANULE NON ALIGNÉE

(30) Priority: 06.02.2019 IT 201900001685
(43) Date of publication of application: 15.12.2021
(73) Proprietor: Sol-Millennium Swiss R&D Center SA, 6900 Lugano (CH)
(72) Inventor: DE ZOLT, Dario, 21054 Fagnano Olona 54 (VA) (IT); LAGANA', Matteo, 22030 Longone Al Segrino (CO) (IT)
(74) Representative: Ripamonti, Enrico
(86) International application number: PCT/IB2020/050495
(87) International publication number: WO 2020/161553

(56) References cited:
- WO-A1-95/19193
- WO-A1-2014/201709
- WO-A1-2019/053568
- US-A- 5 256 152
- US-A- 5 273 540
- US-A- 5 997 507
- US-A1- 2004 267 200

## Description

The subject of the present invention is a medical device according to the preamble of claim 1.

Many medical devices for percutaneous or venous access are known, for the purpose of administering or collecting fluids to and from a patient. Examples of these medical devices are microperfuser needles, fistula needles and catheter needles.

Medical devices of this type comprise a rigid component with a cutting free end, usually a metal cannula, which is secured on a first end of a cannula-holder. The latter is inserted within a body of the device, and has a second end to which there is secured a (plastic) tube in which the fluid supplied to, or taken from, the patient flows. The tube (and a connector connected thereto) permits the transfer of the bodily fluids from and to corresponding accessories, for example specimens for collection under vacuum. A further possible configuration on the other hand is that which can be assimilated to a catheter needle, where however the flexible tube is not present (but wherein this catheter needle comprises other components known to persons skilled in the art).

In one of the aforementioned possible configurations, the cannula pierces the skin of the patient, and puts the other components of the device, which are usually made of plastic, into hydraulic communication with the site of collection or infusion.

In some of these devices, for example in fistula or microperfuser needles, there are very flexible plastic components (such as fins made of soft plastic) which contribute to easy and correct insertion of the cannula in the injection site. Usually, these components or flexible fins are associated with the body of the device, and can be coupled in the vertical position in order to facilitate the insertion of the cannula in the patient.

The fins are also provided with a specific surface finish which facilitates grasping thereof, and improves the breathability of the skin when these fins, which are widened relative to the cannula, are secured on the patient by means of appropriate removable attachment elements.

With these devices there is the problem associated with possible contact with the cannula (which has a cutting tip) by a health operator after the device has been used in the patient. This can be the source of transmission of infectious diseases which can also be very serious and debilitating, such as AIDS and viral hepatitis for example. For this reason, medical devices of the aforementioned type are known which are provided with safety systems in order to prevent accidental punctures.

For example, the use is known of a protective tubular body made of sufficiently rigid plastic material, which is advanced manually on the cannula after use, as far as a blocking position which protects the tip of the metal cannula. These systems are classified as being of the active type (because they carry out a specific operation, in addition to the normal procedure of use of the device, in order to activate the safety).

According to other devices, the movement of the protective plastic body, or vice versa the retraction of the cannula, is caused by an appropriate automatic mechanism which can be activated deliberately by the user. Often, these systems use as a movement driver a preloaded resilient element, such as a resilient spring with compression or with traction.

One of these devices provided with a safety system is described in EP1306097. This known solution comprises on the cannula-holder a projecting flexible arm which extends from an aperture in the body of the device, when the device is in the position in which the cannula is inserted in the body of the patient. This arm has an end in the vicinity of the cannula-holder in the form of a step which is supported on an edge of the aperture in the body of the device.

The cannula-holder is subjected to the thrust of a compression spring, which tends to move the cannula-holder within the body of the device. This movement is however impeded by the cooperation of the end in the form of a step of the aforementioned projecting flexible arm, until, by means of a suitably shaped means, the arm is pressed in a direction which is orthogonal relative to the axis of the cannula, towards the interior of said aperture. In this case, the end with a step is thrust into the body of the device and separates from the edge of the aperture, thus allowing the spring to move the cannula-holder into the interior of the body of the device, with the consequent total return of the cannula within said body.

A similar solution is described in WO2016007438.

It is known however that the systems or solutions provided with resilient elements which act on the cannula-holder can have disadvantages caused by the excessive speed of return of the cannula, such as, for example, excessively rapid detachment of the cannula from the patient, with the possibility of generation of small spurts of bodily fluids, and with possible pain for the patient, a high level of noise, and vibration at the end of the course of the resilient element which blocks the tip.

In addition, in these known solutions, there is also the possibility that the movement of the cannula can be activated in an undesirable manner during the introduction of the cannula itself into the body of the patient at any other time when this actuation is not desired.

WO 2014/201709 describes a medical device according to the preamble of the main claim.

In this prior art, the medical device has a tubular body with an internal cavity in which a supporting cannula-holder can move, as well as a cannula at a distal end thereof.

At a proximal end, the cannula-holder has a radial projection which extends from the body of the device, and can move within a sliding guide provided in this body, under the action of a spring which acts, inside said body, on the distal end of the cannula-holder.

The radial projection of the cannula-holder co-operates with a proximal end hook of an oscillating unit associated with the body of the device. When said cooperation between the hook and the projection exists, the cannula-holder is blocked in the body of the device.

On the other hand, when the hook is raised from the radial projection, the spring thrusts the cannula-holder such as to slide within said body, so as to give rise to the return into the body of the cannula originally placed on the exterior of said body in order to be used.

The prior art in question thus describes cooperation of a substantially on-off type between said proximal hook and the projection. In fact, by acting on a distal end of said oscillating unit, the hook is raised from the projection, and substantially immediately the spring thrusts the cannula-holder within the body of the device.

This solution has the significant disadvantage of being able to have undesirable release of the cannula-holder in the phase of introduction of the cannula itself into the vein, with consequent return of the cannula into the body of the medical device, and the impossibility of using the device.

This is analogous to the problems caused by the above-described known solutions and systems.

A medical safety device of the above-described type is known from a previous patent application by the same applicant i.e. from WO 2019/053568 (and which forms the pre-characterising part of the main claim of the present document), wherein a spring can recall the cannula after use, to within the body of the device. However, according to this known solution, means are present for release of the movement of the cannula, which means cooperate with corresponding blocking means, and do not intervene immediately, releasing the movement of the cannula-holder and the cannula with respect to the body of the device, when an operator or user acts on said means for release; this intervention, i.e. the release of the movement of the cannula, takes place after a first relative axial movement between said release and blocking means. This prevents the movement of the cannula from taking place unintentionally, for example during the stage of introduction of the cannula itself into the vein of the patient, as a result of the simple touching of these release means by the doctor or health operator who is carrying out this operation.

In the above-described solution, the cannula-holder is produced such as to be flexible (or yielding), and this means that a user must also generate a force on the release means in order to bring this cannula-holder into a deformed condition in which said means for release can permit the effective movement of the cannula-holder.

The objective of the present invention is to provide a medical safety device of the aforementioned type, but which is a valid alternative compared with the similar known solutions, and which at the same time can also facilitate the introduction of the cannula within the body of a patient (for example into one of his veins).

In particular, the objective of the present invention is to provide a medical device of the said type wherein the cannula-holder is not flexible, such as to allow an operator to generate a restrained force on the means for release, in order to obtain an initial controlled movement of the cannula-holder, without the free movement thereof being released, and in order to obtain subsequent release of this movement, such as to permit the return of the cannula into this body.

Another objective of the invention is to provide a device of the said type which also provides a high level of safety, not only because of the fact that the tip of the cannula cannot come into contact with a health operator, but also because there is a reduced possibility that bodily fluids can exit from the protection element and come into contact with the health operator.

A further objective of the present invention is to provide a safety device of the aforementioned type which operates when, and only when, it is wished to avoid activation thereof (as far as safety is concerned) which is altogether undesirable.

Another objective is to provide a safety device of the aforementioned type wherein the tip of the cannula is under no circumstances accessible to an operator after it has been extracted from the vein of the patient, it has been taken to a safety position of the body of the catheter device, and wherein this tip can not even be made accessible deliberately after this extraction.

Another objective is to provide a safety device of the aforementioned type which does not lose its protection characteristics in the long term, with said characteristics remaining unchanged even for a long time after the device itself has been produced.

A further objective is to provide a safety device which has a limited number of components, and is simple to produce and assemble, such as to obtain a high level of reliability in use and restricted costs.

These objectives and others, which will become apparent to persons skilled in the art, are achieved by a medical device according to claim 1.

For greater understanding of the present invention, purely by way of non-limiting example, the following drawings are appended, in which:
figure 1 shows a lateral view of a safety device according to the present invention produced in the form of a microperfuser needle;

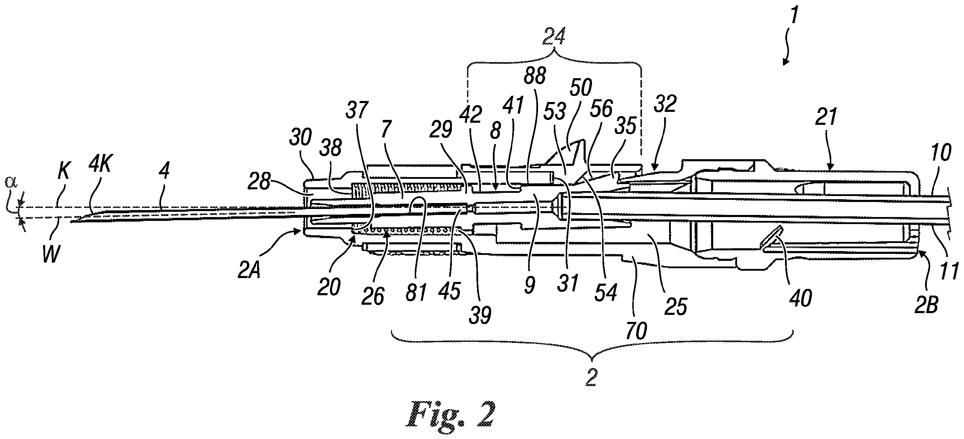

figure 2 shows a longitudinal cross-section of the safety device in figure 1;
figures 3A-3B show views in cross-section of the safety device according to the invention in the various phases of extraction of a cannula from the body of a patient;
figure 4 shows a view in perspective, from one side, of a catheter needle obtained according to the invention;
figure 5 shows a longitudinal cross-section of the catheter needle in figure 4;
figure 6 shows a cross-section similar to figure 5, but of the catheter needle after use.

With reference to said figures, a medical device according to the invention is generally indicated as 1, and comprises a tubular body 2 cooperating with fins 3 (which can be separate from the body 2, or integral with this body) and from which body a cannula 4 extends (from a hole or aperture 28 present at a first end or distal end 2A thereof). When the device is not in use, the cannula is covered by a removable protection element (not shown). As will be described, the cannula 4 is retractable into the body 2 after use.

The cannula 4 is secured in a manner which in itself is known inside a first part or end (distal) part 7 of the cannula-holder 8 (which is mobile in the body 2 when the cannula is retracted), with a second end or end (proximal) part 9 with which a conventional tube 10 is integral. This tube 10 permits transfer of the bodily fluids from and to appropriate known accessories. The cannula-holder 8 is made of rigid or substantially rigid material (i.e. it has its own rigidity associated with the plastic material from which it is constituted, or due to a particular geometric configuration without weaknesses) and does not bend (significantly) along its longitudinal axis. This cannula-holder therefore does not have yielding parts, like the cannula-holder described in WO 2019/053568, which parts are such as to be able to be deformed under the action of the force imposed by the release units during the activation of the safety device.

The body 2 can be in a single piece or, as in the figures, it can be defined by a plurality of components assembled to one another: this body 2 comprises a closed second end or proximal end 2B. In case of a microperfuser needle, the end 2B is provided with a through hole 11 for the exit of the tube 10 from the body 2, but it can also prevent the exit of the cannula-holder 8 from this body.

This tube 10 need not be present in the case when the medical device is a catheter needle.

It will be appreciated that other arrangements can be provided which can prevent the exit of the cannula-holder 8 from this second end 2B when the cannula is retracted into the body 2, such as, for example, an appropriate narrowing or permanent deformation of the second end 2B obtained during the assembly of the device 1, and after the insertion of the cannula-holder 8 in the body 2.

In the embodiment in the figures, the body 2 is produced by means of two components or casings 20 and 21, i.e. a first, distal casing 20 (provided with the hole 28), and a second casing 21 (or proximal casing, from which the tube 10 extends). On this body 2 and in particular on the first casing 20 there is present a slider 24 which can activate the movement of return of the cannula 4 into the body 2 (in the case in the figures, in the first casing 20) within an internal cavity or chamber 25 of this body. This movement is generated by a compression spring 26 disposed between the first end 2A of the first casing 20 and a collar 29 provided on the first end part 7 of the cannula-holder 8.

In the case in the figures, the internal cavity or chamber 25 is defined by both the casings 20 and 21.

The first casing 20 is made of rigid material (for example polypropylene or other materials suitable for the purpose, such as an MABS), and can also be transparent. This casing 20 advantageously has an internal guide (not shown in the figures), preferably with a variable cross-section, in the vicinity of the first end 2A. This guide permits the guided retraction of the cannula-holder 8 into the chamber 25, in particular in the initial movement phase. Other guides (not shown) which are present in the cavity or chamber 25 assist the movement or withdrawal of the cannula-holder 8 into the body 2, and impede its rotation.

The first casing or distal casing 20 also has a window 31 with a side 32 defined by an inclined plane. This side or inclined plane 32 (which acts as a unit for blocking of the cannula-holder) projects within the cavity or chamber 25, and interferes with a blocking tooth 35 of the cannula-holder 8, as will be described.

Finally, the first casing 20 has a cylindrical front projection 30 which delimits the hole 28, and can accommodate the element which protects the cannula 4, as well as ensure covering of a cutting tip 4K of the cannula after the activation of the mechanism for recall into the body 2 (or safety mechanism), with consequent return of the cannula within this body 2. Said recall mechanism comprises the spring 26, the slider 24, the side or inclined plane 32 and the cannula-holder 8 itself.

In the vicinity of this front projection 36, within the chamber or cavity 25, there is present a step 37 on which a first end 38 of this spring is supported, with its second end 39 being supported on the collar 29 of the cannula-holder 8.

Inside the chamber or cavity 25, the second casing 21 (or proximal casing) can comprise a projecting portion or fin 40, which is resiliently deformable in order to permit the movement of the cannula-holder 8 towards the second end 2B of the body 2, when thrust by the spring 26. As will be described hereinafter, this fin 40 can cooperate with a step 41 of an annular recess 42 provided in the cannula-holder 8, which recess separates the parts 7 and 9 from the cannula-holder.

The casings 20 and 21 are coupled to one another in a known manner, for example by embedding. This coupling can alternatively be provided or strengthened by welding (for example thermal or ultrasound welding), or by gluing or any other known system.

As previously stated, the activation slider 24 is a component of the "recall mechanism" of the cannula 4 into the body 2 (i.e. in the example given, into the first casing 20). This mechanism comprises the spring 26 and the (rigid or substantially rigid) cannula-holder 8, which can adopt a position displaced towards the first end 2A of the body 2 (or of the first casing 20), or towards the second end 2B of this body (or of the second casing 21). In this last position, the cannula 4 is inside said body, and protected against accidental contact.

This cursor 24 comprises a semi-cylindrical (or substantially semi-cylindrical) body 46, which can be positioned, and slide, along the first casing 20. This body 46 has on its exterior a profiled projection 50 which can accommodate the finger of an operator who is using the device 1.

In the interior there is present a protuberance 53 which slides on a part 88 of the cannula-holder 8, parallel to the longitudinal axis K of the body 2. This part 88 will be defined hereinafter as "inert", since the sliding of the protuberance 53 on it does not involve any substantial movement of the cannula-holder 8 within the body 2.

The protuberance 53 is provided with a wall or flank 54 which can co-operate with the blocking tooth 35 of the cannula-holder 8. This form of the slider 24 permits initial relative sliding of the slider 24 itself on the cannula-holder 8 (by means of the sliding of the protuberance 53 on the aforementioned inert part 88), without there being an immediate and consequent movement of the cannula-holder 8 in the casing 20.

It should also be noted that the blocking tooth 35 has an inclined wall 56 on which the wall 54 of the slider 24 can slide at the end of the movement on the part 88 of the cannula-holder 8, with the cooperation between said walls 56 and 54 transforming the axial displacement force of the slider on the casing 20 into a vertical component which detaches this blocking tooth 35 from the inclined side 32 of the first casing 20 (acting as a stop unit for the body 2, which can impede the movement of the cannula-holder 8 until it is required, i.e. until the protuberance 53 moves on the inert part 88) .

Since the cannula-holder is rigid or substantially rigid and not flexible like the cannula-holder described in WO 2019/053568, the axial force generated by an operator on the slider 24 is transferred completely to the cannula-holder, which is substantially immediately displaced into the cavity or chamber 25, substantially rotating therein towards the side of said cavity 25 opposite the one where the slider 24 is present.

This immediacy of the displacement of the cannula-holder 8 into the cavity or chamber 25 does not take place in the case of a flexible cannula-holder. In fact, in the case of a flexible cannula-holder described in WO 2019/053568, part of the activation energy is expended in order to deform the component and obtain the required displacement. On the other hand, in the case of a rigid cannula-holder according to the present invention, this displacement is obtained directly without deformation, and thus, for the same geometries, materials and masses involved, with less expenditure of energy.

Consequently, thanks to the rigidity of the cannula-holder, the present invention permits more direct control of the movement of the slider on the cannula-holder, and the final release of the movement of return of the cannula into the body 2.

As described above, (and unlike what is described in WO 2014/201709), before releasing the blocking tooth 35, the slider 24 must travel a short distance unloaded (i.e. without interacting with the tooth 35, such as to displace it from the inclined side 32), which, together with the cooperation of the inclined planes, the (rigid or substantially rigid) materials used and the friction obtained, contributes towards reducing the risk of accidental activation (because it is difficult for accidental contact with the slider to give rise to activation of the recall mechanism), while still maintaining easy and comfortable activation.

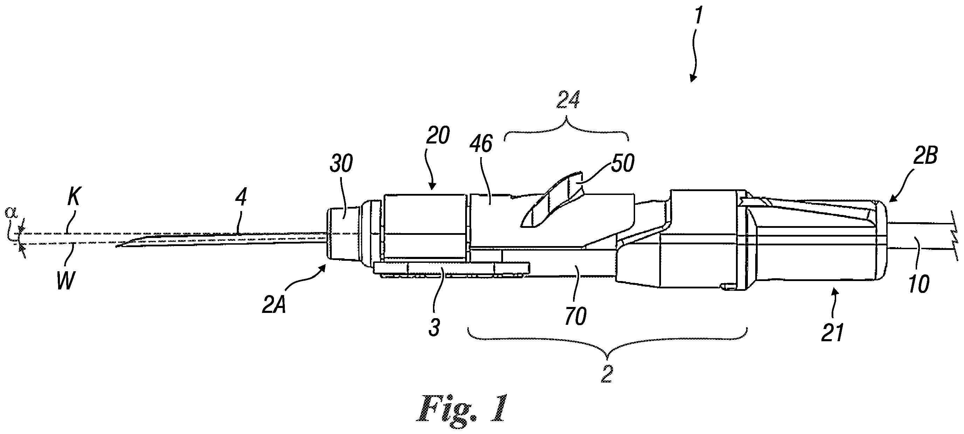

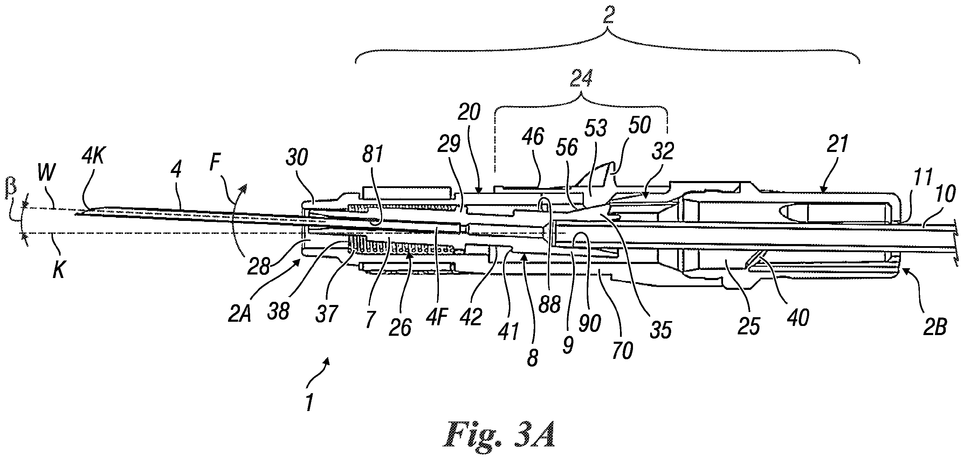

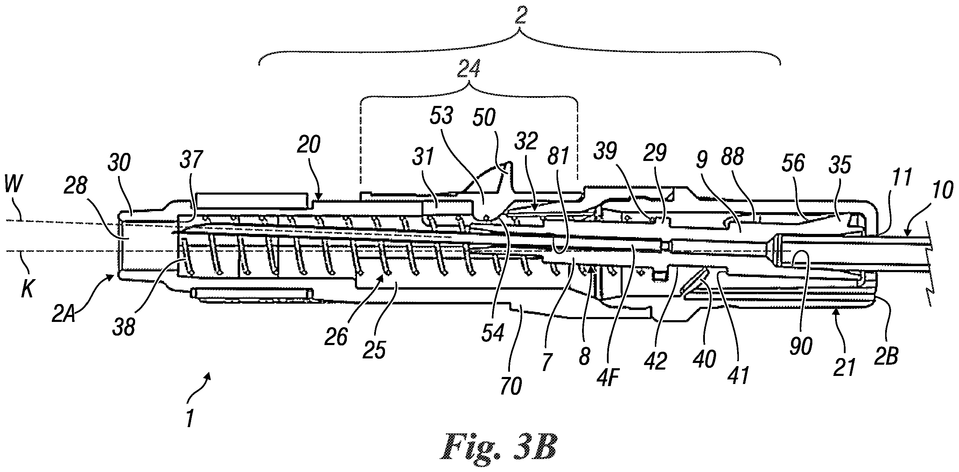

Also according to the invention, the cannula 4, or rather its longitudinal axis W forms together with the longitudinal axis K of the body 2 an angle α contained between -20° and +20°, with negative values considered to be those of the angle measured in an anticlockwise direction relative to the axis W, or rather the angle which this axis W forms with the axis K when the cannula is disposed in the position rotated upwards relative to this axis K (substantially as shown in figures 3A and 3B). The position adopted by the cannula in figure 1 forms a positive angle ex.

This angle can adopt any value in said interval.

This facilitates the movement of the cannula-holder 8 in the chamber or cavity 25 of the body 2.

Preferably, the angle α is contained between +10° and -10°, and advantageously between -3° and +3°. This is in order to reduce the possible perception of vertical movement of the cannula, which the patient could perceive during the activation of the safety device.

It is now assumed that the safety device in the figures is being used.

The use of the invention is as follows: with reference by way of example to use of a microperfuser needle, after having used the device 1 in a manner which in itself is known, by inserting the metal cannula 4 into a vein (or into another suitable site according to the medical procedure in progress), and administering and/or collecting fluids to/from the patient, a folded piece of gauze (or an equivalent) is positioned on the injection site in the vicinity of the cannula 4, in order to prevent bodily fluids from exiting after the withdrawal of the cannula itself. The recall mechanism is activated by means of the axial displacement of the slider 24 on the body 2, with linear movement parallel to the longitudinal axis K of the body 2. This involves initial displacement of the protuberance 53 of the slider 24 towards the inclined wall 56 of the cannula-holder 8, which displacement does not however give rise to any action of thrusting on the blocking tooth 35 of the cannula-holder 8 itself. Thus, this tooth does not slide on the inclined side 32 of the first casing 20 of the body 2 of the device.

Continuing with the displacement, the protuberance 53 slides on the wall 56 of the tooth 35, and begins to press this tooth towards the interior of the chamber 25.

During the movement of the protuberance 53 on the tooth 35, the rigid cannula-holder 8 moves in the interior of the chamber 25 of the device 1, with its proximal part 9 being lowered towards a side 70 of said chamber opposite the window 31. This involves corresponding raising of the first part or distal part 7 of this cannula-holder 8 (arrow F in figure 3A), with consequent displacement of the cannula 4 (which is still on the exterior of the body 2) relative to the axis K. In substance, the axis W of the cannula rotates in the direction of the arrow F relative to the axis K, still remaining distant from this axis at the end of the rotation, and forming therewith an angle β.

When the blocking tooth 35 is displaced below the inclined side 32, the spring 26 can freely thrust the cannula-holder 8 towards the second end 2B of the body 2, making the cannula 4 return completely within this body 2 (i.e. within the internal cavity 25 of the body). In this position, the cannula turns, or could turn, with the axis W in the position which is angled relative to the axis K, and thus have its distal end or tip 4K in a position such that it can not emerge from the aperture 28 of the body 2.

The cannula of the microperfuser needle thus remains securely in the interior of the body 2, therefore making it possible to provide the medical device 1 with the required safety.

In this position (with the cannula-holder 8 completely displaced towards the second end 2B of the body 2), the projecting position or fin 40 is positioned within the recess 42 of the cannula-holder 8. By this means, every movement of the latter towards the opening 28 of the body 2 (whether deliberate or accidental) is opposed and prevented also by the stop of the fin 40 on the step 41 of this recess 42.

It will be appreciated that this recess can be replaced by a projection, for example a collar which projects on the cannula-holder 8, or by any other configuration suitable for the purpose. In both cases, said fin 40 permits the movement of the cannula-holder 8 towards the end 2B of the body 2, when the cannula 4 is recalled into this body 2 by the action of the spring on said cannula-holder.

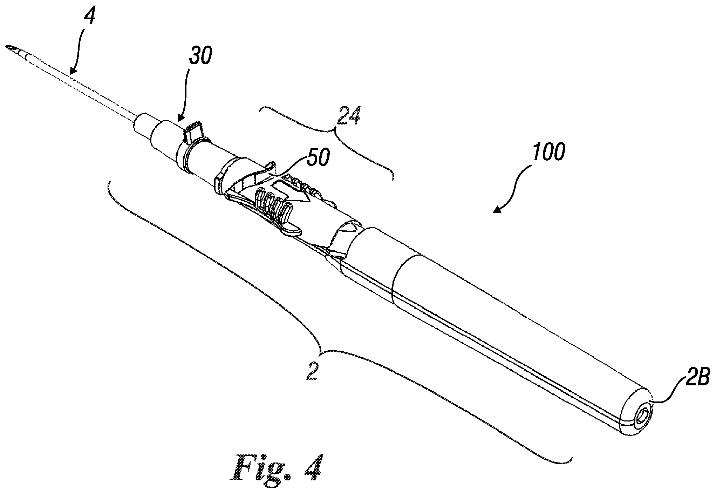

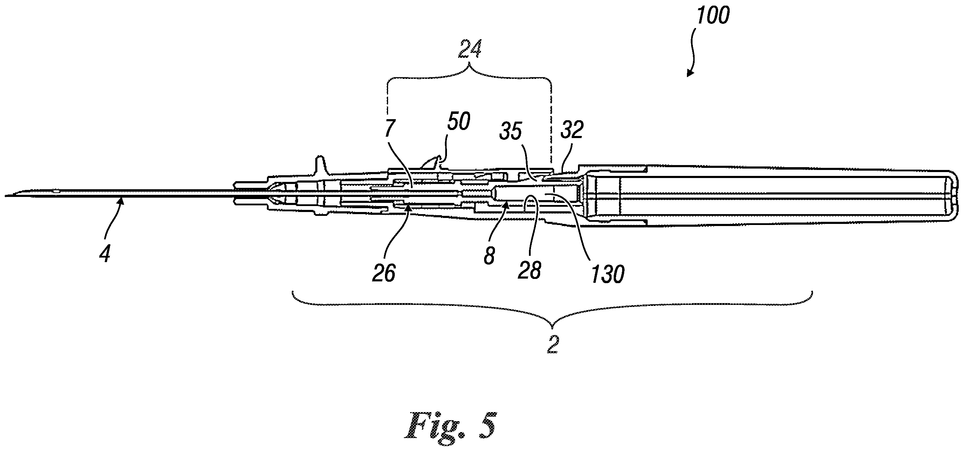

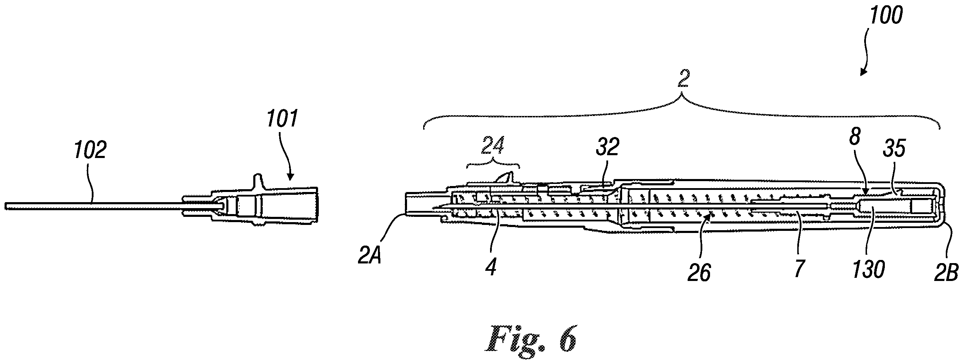

In the case of a catheter needle, or any other similar medical device (not represented in the figures), the movement of the cannula takes place by means of methods identical to those described. This catheter needle is shown in figures 4-6, and is indicated as 100. In these figures, parts which correspond to those of the figures already described are indicated with the same numerical references. This catheter needle 100 has a catheter-holder 101 associated with a catheter 102 which is separable from the tubular body 2, and, after use, can contain the cannula 4 and the respective cannula-holder 8 (subjected to the spring 26).

The use of the catheter needle for the purposes of the return of the cannula within the body 2 is similar to that already described. When the cannula has been returned into the body 2, the spring 26 will retain it within this body, by acting on the cannula-holder 8. This catheter needle usually has a blood reflux chamber 130 at the proximal end 9 of the cannula-holder 8, said reflux chamber being able to have an axis which does not coincide with that of the seat 81 provided at the distal end 7 of the cannula-holder, and being able to co-operate with the cannula 4.

A safety microperfuser needle or catheter needle, or any other medical device similar to the one described, is relatively simple to implement. In fact, the arrangement with an inclined longitudinal axis W (relative to the axis K) of the cannula can be obtained by various means: for example, a seat 81 provided in the distal part 7 of the cannula-holder for a proximal end 4F of the cannula 4 (opposite the tip 4K) can be created inclined (with the same angle of the axis W compared with that K) relative to the longitudinal axis of the cannula-holder 8, which is superimposed on that 15 of the body 2. Or, the cannula-holder 8 is positioned with the longitudinal axis inclined (superimposed on the axis W) within the cavity 25 of the body 2 which contains it; this can be obtained by means of appropriate configuration of the protuberance 53 of the slider 24 and of the surface 56 of the cannula-holder 8 with which this protuberance co-operates, before sliding on the blocking tooth 35.

Alternatively, a seat 90, in the proximal part 9 of the cannula-holder 8 (which in the case of the microperfuser needle can contain the tube 10) can be created in such a way as to incline all of the cannula-holder 8 according to the axis W; the axis of this seat 90 and need not coincide with the axis of the seat 81 for the aforementioned cannula.

Thanks to the invention, a safety medical device is obtained which is simple, as well as easy and reliable to use.

Descriptions have been provided of various embodiments of the invention. Yet others are possible for persons skilled in the art in the light of the foregoing description. This description therefore does not limit this invention, the limits of which are defined by the following claims.

## Claims

1. Medical device (1) for percutaneous or venous access, for the purpose of administering or collecting a fluid to or from a patient, comprising a tubular body (2) having a longitudinal axis (K) and an internal cavity (25), this tubular body having a proximal end (2B) and a distal end (2A) from which a cannula (4) extends in a first position of work, said cannula being supported by a distal end (7) of a cannula-holder (8), said cannula-holder (8) having a proximal end (9), said cannula-holder (8) being able to move within the cavity (25) of said body (2) of the device (1) under the action of a resilient thrust element (26), such as to take the cannula (4) to a second position of work, in which this cannula is inside the tubular body (2), a stop unit (32) which is integral with said body (2) being provided, and being able to prevent said movement of the cannula-holder (8), and actuation means (24) being provided in order to permit this movement, which means are associated with the body (2) of the device (1), and cooperate with actuation counter-means (35) integral with said cannula-holder (8), **characterised in that** said actuation means (24) comprise a protuberance (53) which slides on an inert part (88) of the cannula-holder parallel to the longitudinal axis (K) of the body (2) of the device (1), said sliding taking place at the start of the cooperation between the actuation means (24) and the actuation counter-means, the cannula-holder (8) being non-deformable under the action of the state of force applied during said cooperation, said cannula holder being made of substantially rigid material and being not provided with yielding or bending parts.

2. Medical device according to claim 1, **characterised in that**, in at least one of the positions of work, the cannula (4) has its longitudinal axis (W) disposed inclined relative to the longitudinal axis (K) of the tubular body (2).

3. Medical device according to claim 2, **characterised in that** the cannula (4) has its longitudinal axis (W) which is inclined relative to the axis (K) of the cannula-holder (8) so to form an angle (α) contained between -20° and 20°.

4. Medical device according to claim 3, **characterised in that** the cannula (4) has a longitudinal axis which is inclined relative to the axis (K) of the cannula holder (8) of an angle contained between +10° and -10°, and advantageously between -3° and +3°.

5. Medical device according to claim 2, **characterised in that** the distal end (7) of the cannula-holder (8) is provided with a seat (81) which can contain a proximal end (4F) of the cannula (4), with the axis of said seat coinciding with the longitudinal axis (W) of the cannula (4) .

6. Medical device according to claim 1, wherein this device is a microperfuser needle, **characterised in that** said cannula-holder (8) has, at the proximal end (9), a seat (90) for a tube (10) in which the fluid circulates, said seat (90) being formed such as to position the longitudinal axis of said cannula-holder (8) coinciding with the longitudinal axis (W) of the cannula.

7. Medical device according to claims 5 and 6, **characterised in that** the axis of the seat (81) provided at the distal end (7) of the cannula-holder (8) does not coincide with the axis of the seat (90) which accommodates the tube (10).

8. Medical device according to claim 1, **characterised in that** said tubular body (2) has in its internal cavity (25) thrust means (26) for the cannula-holder (8), which means can position the longitudinal axis thereof coinciding with that of the cannula (W) in the position which is inclined relative to the longitudinal axis (K) of the tubular body (2).

9. Medical device according to claim 1, **characterised in that** said cannula-holder (8) comprises a portion (42) which can cooperate with a flexible fin or equivalent blocking unit (40) present in the cavity (25) of the tubular body (2) where the cannula-holder (8) is mobile.

10. Medical device according to claim 9, **characterised in that** said portion (42) of the cannula-holder (8) is alternatively a recess (42) provided in this cannula-holder (8) or a part which projects from the latter.

11. Medical device according to claim 1, **characterised in that** it is a catheter needle or similar medical device.

12. Medical device according to claims 5 and 11, **characterised in that** the axis of the seat (81) provided at the distal end (7) of the cannula-holder (8) does not coincide with the axis of a reflux chamber which is present at the proximal end of the cannula-holder (8).

## Patentansprüche

1. Medizinische Vorrichtung (1) für perkutanen oder venösen Zugang zum Zweck eines Verabreichens oder Entnehmens eines Fluids an oder von einem Patienten, umfassend einen rohrförmigen Körper (2), der eine Längsachse (K) und einen inneren Hohlraum (25) aufweist, wobei dieser rohrförmige Körper ein proximales Ende (2B) und ein distales Ende (2A), von dem sich eine Kanüle (4) in eine erste Arbeitsposition erstreckt, aufweist, die Kanüle von einem distalen Ende (7) eines Kanülenhalters (8) gehalten wird, wobei der Kanülenhalter (8) ein proximales Ende (9) aufweist, der Kanülenhalter (8) in der Lage ist, sich unter der Wirkung eines elastischen Schubelements (26) innerhalb des Hohlraums (25) des Körpers (2) der Vorrichtung (1) zu bewegen, um die Kanüle (4) in eine zweite Arbeitsposition zu bringen, in der diese Kanüle im Inneren des rohrförmigen Körpers (2) ist, wobei eine Anschlageinheit (32), die fest mit dem Körper (2) verbunden ist, bereitgestellt die in der Lage ist, die Bewegung des Kanülenhalters (8) zu verhindern, und Betätigungseinrichtungen (24), die bereitgestellt sind, um diese Bewegung zu ermöglichen, wobei die Einrichtungen mit dem Körper (2) der Vorrichtung (1) assoziiert sind und mit Betätigungsgegeneinrichtungen (35) zusammenwirken, die fest mit dem Kanülenhalter (8) verbunden sind, **dadurch gekennzeichnet, dass** die Betätigungseinrichtungen (24) einen Vorsprung (53) umfassen, der auf einem inerten Teil (88) des Kanülenhalters parallel zu der Längsachse (K) des Körpers (2) der Vorrichtung (1) gleitet, wobei ein Gleiten zu Beginn des Zusammenwirkens zwischen den Betätigungseinrichtungen (24) und den Betätigungsgegeneinrichtungen erfolgt, wobei der Kanülenhalter (8) unter der Wirkung des Zustands der Kraft, die während der Zusammenwirkung ausgeübt wird, nicht verformbar ist, der Kanülenhalter aus einem im Wesentlichen starren Material gefertigt ist und nicht mit nachgiebigen oder biegsamen Teilen versehen ist.

2. Medizinische Vorrichtung nach Anspruch 1, **dadurch gekennzeichnet, dass** die Längsachse (W) der Kanüle (4) in mindestens einer der Arbeitspositionen geneigt in Bezug auf die Längsachse (K) des rohrförmigen Körpers (2) angeordnet ist.

3. Medizinische Vorrichtung nach Anspruch 2, **dadurch gekennzeichnet, dass** die Längsachse (W) der Kanüle (4) in Bezug auf die Achse (K) des Kanülenhalters (8) geneigt ist, um einen Winkel (α) zu bilden, der zwischen -20° und 20° liegt.

4. Medizinische Vorrichtung nach Anspruch 3, **dadurch gekennzeichnet, dass** die Kanüle (4) eine Längsachse aufweist, die in Bezug auf die Achse (K) des Kanülenhalters (8) um einen Winkel geneigt ist, der zwischen +10° und -10°, vorteilhafterweise zwischen -3° und +3°, liegt.

5. Medizinische Vorrichtung nach Anspruch 2, **dadurch gekennzeichnet, dass** das distale Ende (7) des Kanülenhalters (8) mit einem Sitz (81) versehen ist, der ein proximales Ende (4F) der Kanüle (4) enthalten kann, wobei die Achse des Sitzes mit der Längsachse (W) der Kanüle (4) zusammenfällt.

6. Medizinische Vorrichtung nach Anspruch 1, wobei diese Vorrichtung eine Mikroperfusor-Nadel ist, **dadurch gekennzeichnet, dass** der Kanülenhalter (8) an dem proximalen Ende (9) einen Sitz (90) für einen Schlauch (10) aufweist, in dem das Fluid zirkuliert, wobei der Sitz (90) gebildet ist, um die Längsachse des Kanülenhalters (8) zusammenfallend mit der Längsachse (W) der Kanüle zu positionieren.

7. Medizinische Vorrichtung nach Anspruch 5 und 6, **dadurch gekennzeichnet, dass** die Achse des Sitzes (81), das an dem distalen Ende (7) des Kanülenhalters (8) bereitgestellt ist, nicht mit der Achse des Sitzes (90) zusammenfällt, der den Schlauch (10) aufnimmt.

8. Medizinische Vorrichtung nach Anspruch 1, **dadurch gekennzeichnet, dass** der rohrförmige Körper (2) in seinem inneren Hohlraum (25) Vorschubeinrichtungen (26) für den Kanülenhalter (8) aufweist, wobei die Einrichtungen die Längsachse davon zusammenfallend mit der der Kanüle (W) in der Position, die in Bezug auf die Längsachse (K) des rohrförmigen Körpers (2) geneigt ist, positionieren können.

9. Medizinische Vorrichtung nach Anspruch 1, **dadurch gekennzeichnet, dass** der Kanülenhalter (8) einen Abschnitt (42) umfasst, der mit einer flexiblen Rippe oder einer gleichwertigen Blockiereinheit (40) zusammenwirken kann, die in dem Hohlraum (25) des rohrförmigen Körpers (2) vorhanden ist, in dem der Kanülenhalter (8) beweglich ist.

10. Medizinische Vorrichtung nach Anspruch 9, **dadurch gekennzeichnet, dass** der Abschnitt (42) des Kanülenhalters (8) alternativ eine Aussparung (42), die in diesem Kanülenhalter (8) bereitgestellt ist, oder ein Teil, das aus letzterem hervorsteht, ist.

11. Medizinische Vorrichtung nach Anspruch 1, **dadurch gekennzeichnet, dass** sie eine Katheternadel oder eine ähnliche medizinische Vorrichtung ist.

12. Medizinische Vorrichtung nach den Ansprüchen 5 und 11, **dadurch gekennzeichnet, dass** die Achse des Sitzes (81), der an dem distalen Ende (7) des Kanülenhalters (8) bereitgestellt ist, nicht mit der Achse einer Rückflusskammer zusammenfällt, die an dem proximalen Ende des Kanülenhalters (8) vorhanden ist.

## Revendications

1. Dispositif médical (1) pour un accès percutané ou veineux, dans le but d'administrer ou de prélever un fluide à ou à partir d'un patient, comprenant un corps tubulaire (2) ayant un axe longitudinal (K) et une cavité interne (25), ce corps tubulaire ayant une extrémité proximale (2B) et une extrémité distale (2A) à partir de laquelle s'étend une canule (4) dans une première position de travail, ladite canule étant supportée par une extrémité distale (7) d'un porte-canule (8), ledit porte-canule (8) ayant une extrémité proximale (9), ledit porte-canule (8) pouvant se déplacer à l'intérieur de la cavité (25) dudit corps (2) du dispositif (1) sous l'action d'un élément de poussée élastique (26), de manière à amener la canule (4) à une deuxième position de travail, dans laquelle cette canule se trouve à l'intérieur du corps tubulaire (2), un dispositif d'arrêt (32) solidaire dudit corps (2) étant prévu, et pouvant empêcher ledit mouvement du porte-canule (8), et des moyens d'actionnement (24) étant prévus pour permettre ce mouvement, lesquels moyens sont associés au corps (2) du dispositif (1), et coopèrent avec des contre-moyens d'actionnement (35) partie intégrante dudit porte-canule (8), **caractérisés en ce que** lesdits moyens d'actionnement (24) comportent une protubérance (53) qui coulisse sur une partie inerte (88) du porte-canule parallèle à l'axe longitudinal (K) du corps (2) du dispositif (1), ledit coulissement s'effectuant au début de la coopération entre les moyens d'actionnement (24) et les contre-moyens d'actionnement, le porte-canule (8) étant indéformable sous l'action de la force appliquée pendant ladite coopération, ledit porte-canule étant constitué d'un matériau sensiblement rigide et ne comportant pas de parties qui se déforment ou se plient.

2. Dispositif médical selon la revendication 1, **caractérisé en ce que**, dans au moins une des positions de travail, la canule (4) dispose de son axe longitudinal (W) disposé de façon inclinée par rapport à l'axe longitudinal (K) du corps tubulaire (2).

3. Dispositif médical selon la revendication 2, **caractérisé en ce que** la canule (4) dispose d'un axe longitudinal (W) incliné par rapport à l'axe (K) du porte-canule (8) de manière à former un angle (α) compris entre -20° et 20°.

4. Dispositif médical selon la revendication 3, **caractérisé en ce que** la canule (4) dispose d'un axe longitudinal incliné par rapport à l'axe (K) du porte-canule (8) d'un angle compris entre +10° et -10°, et avantageusement entre -3° et +3°.

5. Dispositif médical selon la revendication 2, **caractérisé en ce que** l'extrémité distale (7) du porte-canule (8) est pourvue d'un siège (81) pouvant contenir une extrémité proximale (4F) de la canule (4), l'axe dudit siège coïncidant avec l'axe longitudinal (W) de la canule (4).

6. Dispositif médical selon la revendication 1, dans lequel ce dispositif est une aiguille de microperfuseur, **caractérisé en ce que** ledit porte-canule (8) présente, à l'extrémité proximale (9), un siège (90) pour un tube (10) dans lequel circule le fluide, ledit siège (90) étant formé de manière à positionner l'axe longitudinal dudit porte-canule (8) de manière à ce qu'il coïncide avec l'axe longitudinal (W) de la canule.

7. Dispositif médical selon les revendications 5 et 6, **caractérisé en ce que** l'axe du siège (81) prévu à l'extrémité distale (7) du porte-canule (8) ne coïncide pas avec l'axe du siège (90) qui reçoit le tube (10).

8. Dispositif médical selon la revendication 1, **caractérisé en ce que** ledit corps tubulaire (2) possède dans sa cavité interne (25) des moyens de poussée (26) destinés au porte-canule (8), lesquels moyens peuvent positionner son axe longitudinal coïncidant avec celui de la canule (W) dans la position inclinée par rapport à l'axe longitudinal (K) du corps tubulaire (2).

9. Dispositif médical selon la revendication 1, **caractérisé en ce que** ledit porte-canule (8) comprend une partie (42) qui peut coopérer avec une ailette flexible ou une unité de blocage équivalente (40) présente dans la cavité (25) du corps tubulaire (2) où le porte-canule (8) est mobile.

10. Dispositif médical selon la revendication 9, **caractérisé en ce que** ladite partie (42) du porte-canule (8) est soit une cavité (42) ménagée dans ce porte-canule (8), soit une partie faisant saillie de ce dernier.

11. Dispositif médical selon la revendication 1, **caractérisé en ce qu'**il s'agit d'une aiguille de cathéter ou d'un dispositif médical similaire.

12. Dispositif médical selon les revendications 5 et 11, **caractérisé en ce que** l'axe du siège (81) prévu à l'extrémité distale (7) du porte-canule (8) ne coïncide pas avec l'axe d'une chambre de reflux présente à l'extrémité proximale du porte-canule (8).
